# EUROPEAN PATENT APPLICATION

(11) **EP 4 620 484 A1**
(43) Date of publication of application: **24.09.2025**
(21) Application number: 23890872.7
(22) Date of filing: 16.11.2023
(51) Int. Cl.: A61K 45/00, A61K 31/5377, A61P 3/04, A61P 3/10, A61P 35/00, A61P 29/00

(54) **METHOD FOR MODULATING KINASE ACTIVITY AND USE THEREOF**

(30) Priority: 16.11.2022 CN 202211436260
(71) Applicant: Center for Excellence in Molecular Cell Science, Chinese Academy of Sciences, Shanghai 200031 (CN); Shanghai Jiao Tong University, Shanghai 200240 (CN)
(72) Inventor: GAO, Daming, Shanghai 200031 (CN); ZHANG, Ao, Shanghai 200240 (CN); WANG, Kaihua, Shanghai 200031 (CN); DING, Chunyong, Shanghai 200240 (CN)
(74) Representative: Mewburn Ellis LLP
(86) International application number: PCT/CN2023/132128
(87) International publication number: WO 2024/104443

(57) **Abstract**

A use of an active ingredient that carries out targeted regulation of metabolite expression levels or closes off a binding site of an LKB1 protein and a metabolite for preventing and/or treating an AMPK kinase-mediated metabolic-related disease. The metabolic-related disease is selected from obesity, diabetes, tumors, inflammatory diseases, or a combination thereof.

## Description

### TECHNICAL FIELD

The present invention relates to the field of biochemistry, specifically, the present invention relates to a method for modulating kinase activity and use thereof.

### BACKGROUND

AMPK (Adenosine 5 '- monophosphate (AMP) - activated protein kinase) is an AMP dependent protein kinase and an important signaling molecule in the regulation of energy metabolism in vivo, and it mainly functions as a protein kinase by phosphorylating downstream protein substrates to exercise its physiological functions. Therefore, the regulation of AMPK kinase activity is the key way to intervene AMPK kinase and treat metabolic related diseases (obesity, diabetes, tumors, inflammatory diseases, etc.). AMPK exists in the body as a trimeric complex and functions as a kinase, including an α-catalytic subunit, a β-regulatory subunit, and a γ-regulatory subunit. Different species may encode multiple highly homologous α, β, and γ subunits, for example, the human genome encodes two highly homologous AMPK α-catalytic subunits (named α1 and α2, respectively). The activation of AMPK kinase depends on the phosphorylation of threonine residues (underlined) in its α-catalytic subunit protein FLRTSC motif (e.g. T183 site of human AMPK α1 protein and T172 site of AMPK α2 protein), and LKB1 and CaMKK β are known to be two important upstream kinases that phosphorylate this site.

LKB1 kinase (Liver kinase B1) is also known as STK11 (Serine-Thranine Kinase 11), or PJS protein (Peutz-Jeghers syndrome, PJS). LKB1 and its homologous proteins catalyze the phosphorylation of multiple kinases, including AMPK kinase α-catalytic subunit, by forming complexes with other proteins (MO25, STRAD1, etc.), thereby regulating physiological activities such as cellular metabolism, proliferation, and autophagy. Therefore, LKB1 is an important disease regulatory target. It has been reported that LKB1 may perceive physiological signals such as cellular metabolic status, but the specific mechanism is still unclear. In previous studies, we discovered metabolic intermediates of the glycolytic metabolic pathway, including 3-phosphoglycerate (chemical formula C₃H₇O₇P, CAS # 820-11-1), 2-phosphoglycerate (chemical formula C₃H₇O₇P, CAS # 2553-59-5), pyruvate (also known as 2-oxopropionic acid, acetoformic acid, pyruvic acid or pyruvate, chemical formula C₃H₄O₃, CAS # 127-17-3), and their corresponding salt forms. These intermediates can directly bind to LKB1 kinase or LKB1 kinase complex and inhibit the phosphorylation of AMPK kinase α-catalytic subunit by LKB1 kinase. Therefore, LKB1 kinase or LKB1 kinase complex can directly perceive intermediate products of glucose metabolism and affect the activation of AMPK kinase by LKB1 kinase or LKB1 kinase complex. Therefore, there is an urgent need in this field to develop new active ingredients capable of carrying out targeted regulation of metabolite expression levels, which is then used in the preparation of pharmaceutical compositions, and the pharmaceutical composition is then used for the prevention and/or treatment of AMPK kinase mediated metabolic-related diseases, such as obesity, diabetes, tumors, inflammatory diseases, etc.

### SUMMARY OF THE INVENTION

The purpose of the present invention is to provide a use of a novel active ingredient capable of carrying out targeted regulation of metabolite expression levels in the preparation of a pharmaceutical composition for the prevention and/or treatment of AMPK kinase mediated metabolic-related diseases.

In the first aspect of the present invention, provided is a use of an active ingredient that carries out targeted regulation of metabolite expression levels or closes off a binding site of an LKB1 protein and a metabolite in the preparation of a pharmaceutical composition, and the pharmaceutical composition is used for preventing and/or treating an AMPK kinase-mediated metabolic-related disease; and the metabolite is selected from the group consisting of: 3-phosphoglycerate, 2-phosphoglycerate, pyruvate, or a combination thereof.

In another preferred example, the active ingredient is used to downregulate the metabolite.

In another preferred example, the AMPK kinase-mediated metabolic-related disease is selected from the group consisting of: obesity, diabetes, tumors, inflammatory diseases, or a combination thereof.

In another preferred example, the active ingredient is used to downregulate the generation of the metabolite or close off the binding of the metabolite and LKB1 kinase or LKB1 kinase complex.

In another preferred example, the close off is competitive or non-competitive close off.

In another preferred example, the active ingredient is used to activate the LKB1-AMPK pathway.

In another preferred example, the active ingredient is used to inhibit the activity of the metabolic enzyme that produces the metabolites.

In another preferred example, the metabolic enzyme is selected from the group consisting of: phosphoglycerate kinase PGK1, PGK2;
phosphoglycerate mutase PGAM1, PGAM2, PGAM3, PGAM4, PGAM5;
pyruvate kinase PKM1, PKM2, PKRL;
pyruvate dehydrogenase PDHK1, PDHK2, PDHK3, PDHK4,
or a combination thereof.

In another preferred example, the active ingredient is used to activate the metabolic pathway that consumes 3-phosphoglycerate, 2-phosphoglycerate, and/or pyruvate or salts thereof.

In another preferred example, the active ingredient is further used to inhibit the transcriptional expression of the metabolic enzyme, and the metabolic enzyme is used to catalyze the production of 3-phosphoglycerate, 2-phosphoglycerate, and/or pyruvate or salts thereof.

In another preferred example, the active ingredient is selected from the group consisting of: small molecule drugs, antibodies, molecular glues, Protac, delivery carriers containing mRNA, delivery carriers containing antisense nucleic acid sequence RNA, or a combination thereof.

In another preferred example, the active ingredient is compound S33:

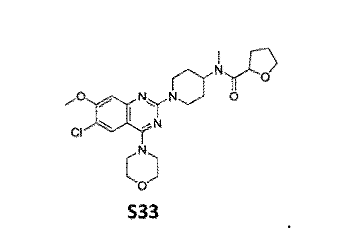

In the second aspect of the present invention, provided is a method for non-therapeutically regulating the LKB1-AMPK pathway in vitro, comprising culturing cells or tissues in the presence of an active ingredient that carries out the targeted regulation of metabolite expression levels;
the metabolite is selected from the group consisting of: 3-phosphoglycerate, 2-phosphoglycerate, pyruvate, or a combination thereof.

It should be understood that, within the scope of the present invention, each of the above technical features of the present invention and each of the technical features specifically described in the following (such as the examples) can be combined with each other to constitute a new or preferred technical solution. Due to space limitations, it will not be repeated herein.

### DESCRIPTION OF THE DRAWINGS

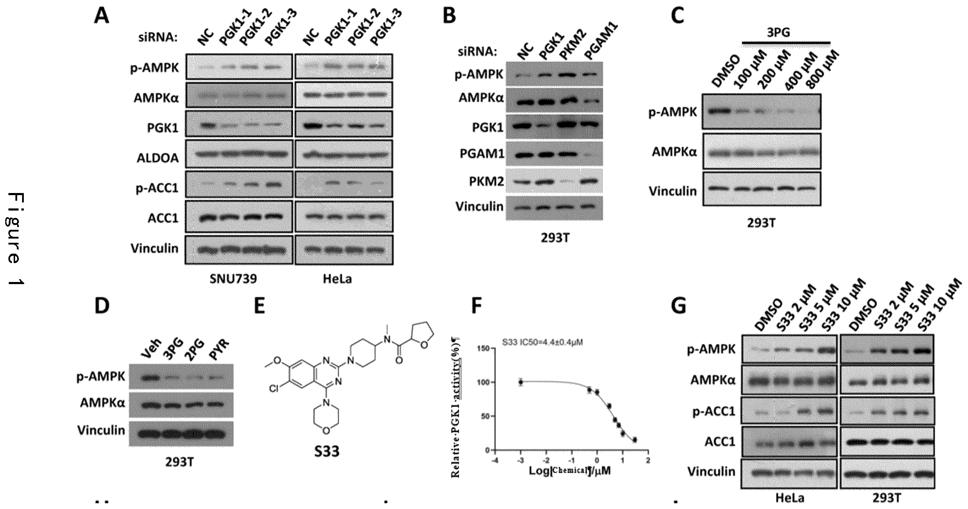

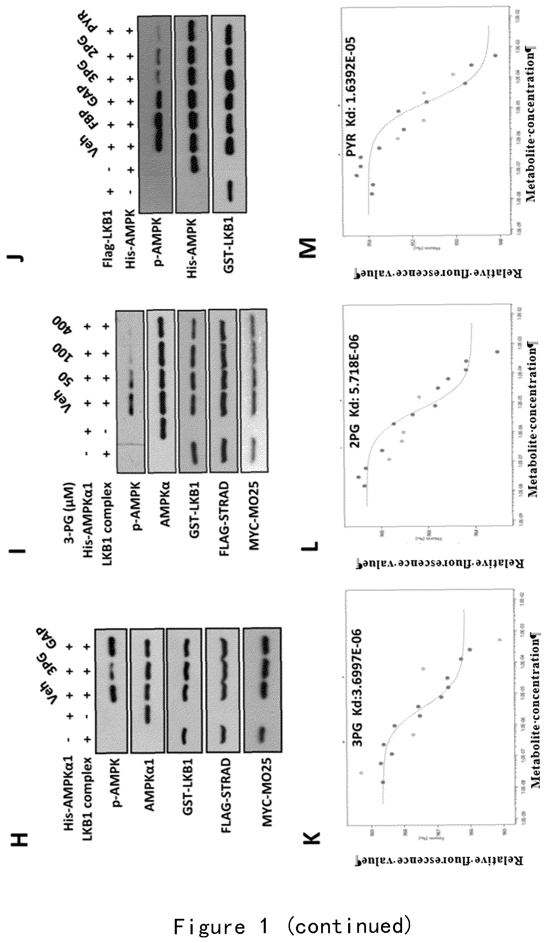

Figure 1A shows the activation of the AMPK signaling pathway in SUN739 hepatocellular carcinoma cell lines and HeLa cell lines transfected with PGK1-targeting siRNA to knock down PGK1, which was detected by Western blot.
Figure 1B shows the activation of AMPK signaling pathway in HEK293T cell lines transfected with siRNA targeting PGK1, PGMA1, PKM2 to knockdown three endogenous metabolic enzymes, which was detected by western blot of collected cells.
Figure 1C shows the activation of AMPK in cultured HEK293T cells subjected to glucose starvation treatment, followed by the treatment of 3-phosphoglycerate (3PG) with different concentrations as shown in the figure, which was detected by western blot of collected cells.
Figure 1D shows the activation of AMPK in cultured HEK293T cells subjected to glucose starvation treatment, followed by the addition of 200 µM of 3-phosphoglycerate (3PG), 2-phosphoglycerate (2PG), and pyruvate (PYR), respectively, which was detected by western blot of collected cells.
Figure 1E shows the structure of compound S33.
Figure 1F shows the enzyme activity curve and calculated IC₅₀ value of PGK1 under the treatment of compound S33.
Figure 1G shows the changes in specific proteins and the changes in AMPK α - catalytic subunit phosphorylation sites in HeLa and HEK293T cell lines under the treatment of compound S33, as well as the phosphorylation of the classical substrate ACC1 downstream of the AMPK pathway.
Figure 1H shows that HEK293T cells were processed to overexpress LKB1 complex related plasmids (GST-LKB1/FLAG-STRAD1/Myc-MO25), and purified by immunoprecipitation to obtain LKB1 complex, and then co-incubated with recombinant His-AMPK protein expressed in Escherichia coli to conduct in vitro kinase experiments, and metabolites 3-phosphoglycerate (3PG) or 3-phosphoglyceraldehyde (GAP) of 100µM each or corresponding solvent control (veh) was simultaneously added, and the effect on AMPK phosphorylation mediated by LKB1 kinase complex was detected by western blot.
Figure 1I shows the effect of 3-phosphoglycerate (3PG) on AMPK phosphorylation mediated by LKB1 kinase complex after adding three concentrations of 3-phosphoglycerate (3PG) separately in an in vitro kinase experiment similar to Figure 1E using the purified LKB1 complex described above, which was detected by western blot.
Figure 1J shows that HEK293T cells were processed to overexpress GST-LKB1, and purified by immunoprecipitation to obtain LKB1 kinase, and then co-incubated with recombinant His-AMPK protein expressed in Escherichia coli to conduct in vitro kinase experiments, and metabolites 3-phosphoglycerate (3PG), 2-phosphoglycerate (2PG), and pyruvate (PYR) of 100 µM each was simultaneously added, and the effect on AMPK phosphorylation mediated by LKB1 kinase was detected by western blot.
Figures 1K to 1M show the dissociation constants (Kd values) between LKB1 protein and metabolites measured and calculated using a microscale thermophoresis instrument after the large-scale purification of LKB1 kinase using HEK293F cells, which was co-incubated with different concentrations of 3-phosphoglycerate (3PG),
2-phosphoglycerate (2PG), and pyruvate (PYR).

### DETAILED DESCRIPTION

After extensive and in-depth research, the inventor has developed for the first time a novel active ingredient that carries out targeted regulation of metabolite expression levels through extensive screening, and used it to prepare pharmaceutical compositions for the prevention and/or treatment of AMPK kinase mediated metabolic-related diseases. The present invention is completed on this basis.

The present invention was further described hereafter in combination with specific examples. It should be understood that these examples are only used to illustrate and not to limit the scope of the invention. The experimental methods without specific conditions in the following examples generally follow the conventional conditions or the conditions suggested by the manufacturer. Unless otherwise stated, percentages and parts are by weight. Unless otherwise specified, the experimental materials and reagents used in the following examples are commercially available.

### General Experimental Method

### 1. Transfection of siRNA

siRNA dry powder synthesized by Shanghai Biotend Biotechnology Co., Ltd was dissolved in DEPC water. Transfection was carried out using X-tremeGene siRNA Transfection Reagent (Roche) according to the instructions. The appropriate cell density for siRNA transfection was around 30%. In our laboratory, Roche X-tremeGene siRNA transfection reagent was used, two 1.5mL RNase free centrifuge tubes were taken, and each added with 250µL Opti-MEM, and 5µL of X-tremeGene siRNA transfection reagent and 6.25µL of siRNA (20µM) were added, respectively. Each was gently blown evenly with a pipette and let it stand at room temperature for 5 minutes. The Opti-MEM containing X-tremeGENE siRNA transfection reagent and the Opti-MEM containing siRNA were mixed, gently blown evenly using pipette, and let it stand for 15 minutes. 293T cells were taken out, the culture medium was removed, and the cells were washed with PBS buffer once, and added with 2mL Opti MEM. The Opti-MEM mixture of plasmid and transfection reagent was slowly added into the cells, and gently shaken to ensure even distribution of siRNA. 5 hours later, 2 mL of DMEM complete culture medium containing serum and antibiotics was replenished to the transfected cells. The cells to be transfected was replaced with normal DMEM cell culture medium. 36 hours later, cells were collected and the total cell protein was extracted. The changes in specific proteins and phosphorylation of specific sites were detected using commercial antibodies through Western blot.

### 2. Metabolite treatment of cells

Cells were subjected to glucose starvation treatment using glucose free culture medium for 2 hours. The corresponding metabolites were added to the culture medium according to the instructions shown in Figure 1C and Figure 1D, so that their concentration in the culture medium reached the labeled level, if there was no labeling, the concentration of metabolites in the culture medium was 200M. After treatment, cells were collected to prepare lysis buffer, and the changes in specific proteins and phosphorylation of specific sites were detected by Western blot.

### 3. Plot of in vitro enzyme activity of PGK1 and compound IC₅₀ inhibition curve

A substrate buffer mixture with a final concentration of 10 mM KH₂PO₄, pH7.0, 2mM GAP, 0.6mM β-NAD, 0.4 mM ADP, 10 mM MgSO₄, 200mM Glycine, and 0.01µg/µL GAPDH was prepared. The purified hPGK1 recombinant protein was diluted to 0.2 ng/µL. Inhibitors were added to the protein and a concentration gradient was set up. Each reaction was added with 100 µl of substrate and a mixture of 100 µl hPGK1 protein and inhibitor, and reacted at 37 °C for 25 minutes. After centrifuged at 10000rpm for 10 minutes at 4 °C, 100 µl of supernatant was taken. In the ATP detection kit (Beyotime S0026), the ATP detection solution was diluted with ATP detection diluent at a ratio of 1:10. In a 96 well blank plate, each well was added with 100 µl of diluted detection solution, and let it stand at room temperature for 5 minutes, added with 100 µl of supernatant from enzyme activity reaction, and reacted for 5 seconds, and a microplate reader was used to read the fluorescence value. The relative enzyme activity was calculated based on fluorescence values and IC50 values were calculated using GraphPad nonlinear regression fitting.

### 4. In vitro kinase experiment

The final reaction system for kinase experiment was: 50mM Tris HCl, 10mM MgCl₂, 1mM DTT, and 100 µM ATP. HEK293T cells were co-transfected with equal amounts of GST-LKB1, FLAG-STRAD, MYC-MO25 plasmids or solely transfected with FLAG-LKB1 plasmid. The cells were collected, processed for immunoprecipitation using GST beads or FLAG beads, washed three times with cell lysis buffer containing NP-40, and washed twice with kinase reaction buffer. The His-AMPK protein was induced and purified using E. coli BL21 competent cells. The kinase was incubated with metabolites at 100 µM at room temperature for 15 minutes before reaction, then added with reaction buffer and His-AMPK protein, and reacted at 30 °C for 30 minutes. The phosphorylation of AMPK protein was detected by Western blot.

### 5. Microscale thermophoresis experiment

LKB1 protein was purified, and fluorescently labeled using NHS dye. 1mM metabolite solution was prepared, and diluted in a 2-fold gradient with a total of 16 gradients. The labeled protein was diluted to 25nM and mixed with the metabolite solution at a ratio of 1:1, which was aspirated using a glass capillary and detected in a microscale thermophoresis instrument NT.115. The data was directly processed using the data acquisition software MO. Control to obtain the dissociation constant.

### 6. Cell Culture

HEK293T cell line, SUN739 hepatocellular carcinoma cell line, and HeLa cell line were all cultured in DMEM medium. All cells were cultured in a 37 °C/5% CO₂ cell culture incubator and passaged every 2-3 days. After passaged for about 30 generations, cells need to be thawed for use.

### Example 1 Study on the regulatory mechanism of PGK1 knockdown on LKB1-AMPK pathway

siRNA dry powder synthesized by Shanghai Biotend Biotechnology Co., Ltd was dissolved in DEPC water. Transfection was carried out using X-tremeGene siRNA Transfection Reagent (Roche) according to the instructions so as to conduct PGK1 knockdown experiment. For each siRNA transfection, two 1.5mL RNase free centrifuge tubes were taken, and each added with 250µL Opti-MEM, and 5µL of X-tremeGene siRNA transfection reagent and 6.25µL of siRNA (20µM) were then added, respectively. Each was gently blown evenly with a pipette and let it stand at room temperature for 5 minutes. The Opti-MEM containing X-tremeGENE siRNA transfection reagent and the Opti-MEM containing siRNA were mixed, gently blown evenly using pipette, and let it stand for 15 minutes. The hepatocellular carcinoma cell line SNU739 and cervical cancer cell line HeLa to be transfected were taken out, the culture medium was removed, and the cells were washed with PBS buffer once, and added with 2mL Opti MEM. The Opti-MEM mixture of plasmid and transfection reagent was slowly added into the 293T cells, and gently shaken to ensure even distribution of siRNA. 5 hours later, 2 mL of DMEM complete culture medium containing serum and antibiotics was replenished to the transfected cells. The transfected cells were replaced with normal DMEM cell culture medium. 36 hours later, cells were collected and the total cell protein was extracted. The changes in specific proteins and phosphorylation of specific sites were detected using commercial antibodies through Western blot.

Small RNA interference (SiRNA) experiments were conducted on phosphoglycerate kinase 1 (PGK1), a key metabolic enzyme that catalyzes the production of 3-phosphoglycerate during glycolysis, in hepatocellular carcinoma cell line SNU739 and cervical cancer cell line HeLa. The western blot results showed that in the case of siRNA transfection mediated PGK1 knockdown, the band signal representing AMPK α-catalytic subunit phosphorylation level (p-AMPK signal, representing the phosphorylation levels of T183 site of AMPK α1 protein and T172 site of AMPK α2 protein) was upregulated, and the phosphorylation level of ACC1, downstream of AMPK, was increased, indicating the activation of the AMPK pathway (Figure 1A). When the metabolic enzymes PGAM1 and PKM2, downstream of PGK1, were knocked down, the AMPK pathway was also activated (Figure 1B).

### Example 2 Study on the regulatory mechanism of 3-phosphoglycerate on AMPK pathway

The function of PGK1 is to catalyze the production of 3-phosphoglycerate (3PG) from 1.3-diphosphoglycerate, PGAM1 catalyzes the production of 2-phosphoglycerate from 3-phosphoglycerate (3PG), and PKM2 catalyzes the production of pyruvate (PYR) from phosphoenolpyruvate. When the expression and function of these metabolic enzymes are inhibited, their corresponding products are decreased. Therefore, we speculate that there may be a mechanism by which AMPK is regulated by metabolite molecules in the glycolytic metabolic pathway, and the activation level of AMPK is related to metabolite content. To verify this hypothesis, we added different doses of exogenous 3-phosphoglycerate (3-PG) to 293T cells subjected to glucose starvation treatment (activation of AMPK signaling pathway). 293T cells under normal culture conditions (37 °C/5% CO₂) were subjected to glucose starvation treatment for 2 hours using DMEM medium without glucose. 3-PG was added to the culture medium according to the instructions in the diagram (as shown in Figure 1C), so as to make its concentration in the culture medium reached the labeled level (ranging from 100-800 µM) (Figure 1C), or until the concentration in the culture medium reached 200 µM (Figure 1D). After treatment, cells were collected to prepare lysate, and the changes in specific proteins and the changes in phosphorylation sites of AMPK α-catalytic subunit were detected by Western blot.

The results showed that 3-phosphoglycerate significantly inhibited intracellular AMPK α-catalytic subunit phosphorylation signaling in a dose-dependent manner (Figure 1C), and exogenous addition of 2-phosphoglycerate (2-PG) and pyruvate (PYR) also had inhibitory effects on AMPK α-catalytic subunit phosphorylation signaling (Figure 1D).

### Example 3 Study on the regulation of PGK1 inhibitor on AMPK pathway

The compound (S33, patent number: ZL201610826187. X) reported in the previous patent is a PGK1 inhibitor (Figure 1E), and we determined the inhibitory activity of compound S33 on PGK1. The recombinant human PGK1 protein was purified using Escherichia coli, and compound S33 was added to establish a concentration gradient. Each reaction was added with 100 µl of substrate and a mixture of 100 µl hPGK1 protein and inhibitor, wherein, hPGK1 recombinant protein was diluted to 0.2 ng/ml, 7 concentration gradients of S33 was established (0, 1, 3, 5, 7, 10, 30µM, respectively). Then a substrate buffer mixture containing 10 mM KH₂PO₄, pH7.0, 2mM GAP, 0.6mM β-NAD, 0.4 mM ADP, 10 mM MgSO₄, 200mM Glycine, and 0.01µg/µL GAPDH was prepared, and reacted at 37 °C for 25 minutes. After centrifuged at 10000rpm for 10 minutes at 4 °C, 100 µl of supernatant was taken. In the ATP detection kit (Beyotime S0026), the ATP detection solution was diluted with ATP detection diluent at a ratio of 1: 10. In a 96 well blank plate, each well was added with 100 µl of diluted detection solution, let it stand at room temperature for 5 minutes, added with 100 µl of supernatant from enzyme activity reaction, and reacted for 5 seconds, and a microplate reader was used to read the fluorescence value. The relative enzyme activity was calculated based on fluorescence values, and IC₅₀ values were calculated using GraphPad nonlinear regression fitting (Figure 1F). The above data indicated that compound S33 was an effective inhibitor of PGK1.

In order to investigate the regulatory effect of PGK1 inhibition on AMPK pathway, different concentrations of S33 (2, 5, and 10 µM, respectively) were added to the culture supernatants of HeLa and 293T cells, and treated for 2 hours. Cell lysate was collected and the changes in specific proteins and in AMPK α-catalytic subunit phosphorylation sites, as well as the phosphorylation of the classical substrate ACC1, downstream of the AMPK pathway, were detected by western blot.

The results showed that PGK1 inhibitor S33 could upregulate the AMPK α-catalytic subunit phosphorylation and ACC1 phosphorylation in a dose-dependent manner. Therefore, the inhibition of PGK1 enzyme activity caused by the compound can activate the AMPK pathway (Figure 1G).

### Example 4 Study on the regulatory mechanism of 3-phosphoglycerate on LKB1-AMPK pathway

Given that LKB1 is a key upstream kinase that phosphorylates and activates the AMPK α subunit, we speculate that the aforementioned metabolites may affect the phosphorylation activation of AMPK α subunit by LKB1 kinase or LKB1 kinase complex. To verify this hypothesis, GST-LKB1, FLAG-STRAD1, and MYC-MO25 were co-transfected into 293T cells (2 µg of each expression plasmid, and transfected into about 7x10⁶ cells). After 48 hours, cells were collected and lysed, and then the LKB1-STRAD1-MO25 kinase complex was subjected to glutathione-agarose bead affinity purification, and co-incubated with the recombinant His-AMPK α1 subunit expressed in E. coli for in vitro kinase experiments. The final reaction system for kinase experiment was: 50 mM Tris HCl, 10 mM MgCl₂, 1 mM DTT, 100 µM ATP. At the same time, metabolites such as 3-phosphoglyceraldehyde (GAP) or 3-phosphoglycerate (3-PG) were added to 100 µM according to the diagram (as shown in Figure 1H), or to a concentration of 50-400 µM as shown in the diagram (as shown in Figure 1I). The kinase complex was pre-incubated with metabolites at room temperature for 15 minutes before reaction. Then the reaction buffer and His-AMPK protein were added, and reacted at 37°C for 30 minutes. The phosphorylation of AMPK protein was detected by Western blot.

The results showed that 3-phosphoglycerate strongly inhibited the phosphorylation of AMPK α1 by LKB1 kinase complex, while 3-phosphoglyceraldehyde had no such effect (Figure 1H). And 3-phosphoglycerate inhibits the phosphorylation of AMPK α1 by LKB1 kinase complex in a dose-dependent manner (Figure 1I).

### Example 5 Inhibition of metabolites on phosphorylation of AMPK α1 kinase by LKB1 kinase in vitro

To further investigate whether the three metabolites of 3-phosphoglycerate (3-PG), 2-phosphoglycerate (2-PG), and pyruvate (PYR) directly act on LKB1 kinase and affect its phosphorylation activation of downstream AMPK kinase, we prepared LKB1 kinase (excluding STRAD1 and MO25) by 293T cells transfection and immunoprecipitation, and co-incubated it with recombinant His-AMPK α1 subunit expressed in E. coli for in vitro kinase experiments. The final reaction system for the kinase experiment was 50 mM Tris-HCl, 10 mM MgCl₂, 1 mM DTT, and 100 µM ATP. At the same time, metabolites of the same concentration (100µM) were added according to the diagram (as shown in Figure 1J): 1,6-fructose diphosphate (FBP), 3-phosphoglyceraldehyde (GAP), 3-phosphoglycerate (3-PG), 2-phosphoglycerate (2-PG), and pyruvate (PYR). The kinase complex was pre-incubated with metabolites at room temperature for 15 minutes before reaction. Then the reaction buffer and His-AMPK protein were added, and reacted at 37°C for 30 minutes. The phosphorylation of AMPK protein was detected by Western blot.

The results showed that 100µM of 3-phosphoglycerate, 2-phosphoglycerate (2-PG), and pyruvate (PYR) could significantly inhibit the phosphorylation of AMPK α1 by LKB1 kinase, while 1,6-fructose diphosphate (FBP) and 3-phosphoglyceraldehyde (GAP) had no such effect (Figure 1J).

### Example 6 Study on the regulatory mechanism of three metabolites on LKB1-AMPK pathway

To further investigate whether LKB1 directly interacts with the three metabolites of 3-phosphoglycerate, 2-phosphoglycerate (2-PG), and pyruvate (PYR), we prepared LKB1 kinase protein through large-scale transfection-affinity purification in 293T cells and molecular sieving. Furthermore, microscale thermophoresis experiment was conducted with the above three metabolites at different concentrations to calculate the binding between LKB1 protein and metabolites. Firstly, 1mM metabolite solution was prepared and gradually diluted in a 2-fold gradient with a total of 16 concentration gradients. Then LKB1 protein was purified, and fluorescently labeled using NHS dye. The labeled protein was diluted to 25nM and mixed with the metabolite solution at an equal volume ratio of 1: 1. The mixture was aspirated using a glass capillary and detected in a microscale thermophoresis instrument NT.115. The data was directly processed using the data acquisition software MO. Control to obtain the dissociation constant.

The results showed that LKB1 protein had strong interactions with three metabolites of 3-phosphoglycerate (3-PG), 2-phosphoglycerate (2-PG), and pyruvate (PYR), and their equilibrium dissociation constants (Kd) were calculated separately (Figure 1K-Figure 1M).

Based on the above results, we believe that the three metabolites of 3-phosphoglycerate, 2-phosphoglycerate, and pyruvate directly bind to LKB1, thereby inhibiting the activation of AMPK α subunit by LKB1 kinase. Therefore, strategies such as targeting metabolic enzymes that produce the aforementioned metabolites to modulate the content of the aforementioned metabolites or closing off the binding sites of LKB1 protein and these metabolites, could serve as effective means to regulate AMPK kinase activity for the modulation of physiological functions and the treatment of diseases.

All documents mentioned in the present invention are cited as references in the present invention, just as each document is individually cited as a reference. In addition, it should be understood that, after reading the above teaching content of the present invention, those skilled in the art can make various changes or modifications to the present invention, and these equivalent forms also fall within the scope defined by the appended claims of the present invention.

## Claims

1. A use of an active ingredient that carries out targeted regulation of metabolite expression levels or closes off a binding site of an LKB1 protein and a metabolite, wherein in the preparation of a pharmaceutical composition, and the pharmaceutical composition is used for preventing and/or treating an AMPK kinase-mediated metabolic-related disease; and the metabolite is selected from the group consisting of: 3-phosphoglycerate, 2-phosphoglycerate, pyruvate, or a combination thereof.

2. The use according to claim 1, wherein the AMPK kinase-mediated metabolic-related disease is selected from the group consisting of: obesity, diabetes, tumors, inflammatory diseases, or a combination thereof.

3. The use according to claim 1, wherein the active ingredient is used to downregulate the generation of the metabolite or close off the binding of the metabolite and LKB1 kinase or LKB1 kinase complex.

4. The use according to claim 3, wherein the close off is competitive or non-competitive close off.

5. The use according to claim 1, wherein the active ingredient is used to activate the LKB1-AMPK pathway.

6. The use according to claim 1, wherein, the active ingredient is used to inhibit the activity of the metabolic enzyme that produces the metabolites.

7. The use according to claim 6, wherein the metabolic enzyme is selected from the group consisting of: phosphoglycerate kinase PGK1, PGK2;
phosphoglycerate mutase PGAM1, PGAM2, PGAM3, PGAM4, PGAM5;
pyruvate kinase PKM1, PKM2, PKRL;
pyruvate dehydrogenase PDHK1, PDHK2, PDHK3, PDHK4,
or a combination thereof.

8. The use according to claim 1, wherein the active ingredient is used to activate the metabolic pathway that consumes 3-phosphoglycerate, 2-phosphoglycerate, and/or pyruvate or salts thereof.

9. The use according to claim 1, wherein the active ingredient is further used to inhibit the transcriptional expression of the metabolic enzyme, and the metabolic enzyme is used to catalyze the production of 3-phosphoglycerate, 2-phosphoglycerate, and/or pyruvate or salts thereof.

10. The use according to claim 1, wherein the active ingredient is selected from the group consisting of: small molecule drugs, antibodies, molecular glues, Protac, delivery carriers containing mRNA, delivery carriers containing antisense nucleic acid sequence RNA, or a combination thereof.

11. A method for non-therapeutically regulating the LKB1-AMPK pathway in vitro, wherein comprising culturing cells or tissues in the presence of an active ingredient that carries out the targeted regulation of metabolite expression levels;
the metabolite is selected from the group consisting of: 3-phosphoglycerate, 2-phosphoglycerate, pyruvate, or a combination thereof.
